# EUROPEAN PATENT APPLICATION

(11) **EP 4 163 925 A1**
(43) Date of publication of application: **12.04.2023**
(21) Application number: 21290061.7
(22) Date of filing: 05.10.2021
(51) Int. Cl.: G16H 20/17

(54) **DETERMINING LUMEN FLOW PARAMETERS**

(71) Applicant: Koninklijke Philips N.V., 5656 AG Eindhoven (NL)
(72) Inventor: Florent, Raoul, 5656 AE Eindhoven (NL); Levrier, Claire, 5656 AE Eindhoven (NL); This, Alexandre, 5656 AE Eindhoven (NL); Schmitt, Holger, 5656 AE Eindhoven (NL); Van Der Horst, Arjen, 5656 AE Eindhoven (NL)

(57) **Abstract**

A system (100) for determining flow parameters of a lumen (110) in a hyperemic state induced subsequent to a contrast agent injection (Inj1) into the lumen in a basal state, is provided. The system comprising one or more processors (120) configured to: determine (S 110), based on received angiographic data representing the injected contrast agent and/or received injector data representing the injected contrast agent, a temporal window (THo, THi) representing a duration of the induced hyperemic state; and output (S120) a signal (Sₕ) indicative of the temporal window (TH₀, TH₁).

## Description

### Technical Field

The present disclosure relates to determining flow parameters of a lumen. A system, a computer-implemented method, and a computer program product, are disclosed.

### Background

Coronary Artery Diseases "CAD" refers to pathologies of the coronary arteries and to the deterioration of their ability to transport oxygenated blood to the heart muscle. Ultimately, the lack of oxygen supply results in myocardial ischemia, whose symptoms can include shortness of breath, angina, or even myocardial infarction.

While obstructive CAD is well recognized as a contributing factor to the reduction of blood supply to the heart muscle, there is an increasing number of patients with evidence of ischemia and/or reports of chest pain without obstructive CAD, also referred to as Non-Obstructive Coronary Artery Disease "NOCAD".

Coronary Microvascular Dysfunction "CMVD", or in short, MVD, has been shown to be a contributing factor to myocardial ischemia and myocardial dysfunction, especially in the NOCAD context. As such, there is a great interest in assessing coronary microvasculature.

Several flow parameters have been proposed for use in evaluating the microvasculature. These include the Coronary Flow Reserve "CFR", the Thrombolysis in Myocardial Infarction "TIMI" flow grade, TIMI frame count, and the Index of Microvascular Resistance "IMR" or the Hyperemic Microvascular Resistance index "HMR". The resistance indices are usually recognized as being more specific and informative as compared to CFR or TIMI methods. IMR and HMR are usually measured invasively using a pressure-wire, although more recently, angiography-derived measurements have been investigated.

However, the measurement of many types of flow parameters, such as the examples described above, suffers from the need to induce a hyperemic state in order for them to be measured. This is the case for flow parameters of coronary lumens, as well as lumens that are found elsewhere in the body. As compared to the basal, or resting, state, blood flow is increased in the hyperemic state. Typically, the hyperemic state is induced by the injection of a drug. The injection may be an intra-coronary or intra-venous injection. Drugs such as Adenosine and Papaverine have been used for this purpose. However, the injection of such drugs complicates workflow. Such drugs may also trigger complications in patients, including rhythmic complications, and ventricular tachycardia.

An alternative technique for inducing the hyperemic state is published in a document by Shiode, N. et al., entitled "Contrast-induced Hyperemia as an Alternative to Drug-induced Hyperemia in the Evaluation of the Fractional Flow Reserve in Coronary Lesions", Internal Medicine 2017; 56: pages 253-257, 2017. The FFR is an index for determining whether or not an angiographically equivocal stenosis is of functional significance. This document reports on the diagnostic accuracy of the FFR after hyperemia is induced through the injection of a contrast agent, as compared to the FFR evaluated after intracoronary papaverine, and concludes that the contrast-induced FFR is highly accurate for predicting the papaverine-induced FFR, and that the contrast-induced FFR might be an alternative method for evaluating the function of coronary artery lesions in clinical practice.

However, there remains a need for improvements in determining flow parameters of lumens in the vasculature.

### Summary

According to one aspect of the present disclosure, a system for determining flow parameters of a lumen in a hyperemic state induced subsequent to a contrast agent injection into the lumen in a basal state, is provided. The system comprises one or more processors configured to:
- determine, based on received angiographic data representing the injected contrast agent and/or received injector data representing the injected contrast agent, a temporal window representing a duration of the induced hyperemic state; and
- output a signal indicative of the temporal window.

In so doing, the system provides a reliable indication of when flow parameter measurements can be made in the hyperemic state.

Further aspects, features, and advantages of the present disclosure will become apparent from the following description of examples, which is made with reference to the accompanying drawings.

### Brief Description of the Drawings

Fig. 1 is a schematic diagram illustrating an example of a system 100 for determining contrast agent flow parameters of a lumen, in accordance with some aspects of the present disclosure.
Fig. 2 is a flowchart illustrating an example of a method of determining contrast agent flow parameters of a lumen, in accordance with some aspects of the present disclosure.
Fig. 3 is a schematic diagram illustrating an example of various timing signals for determining contrast agent flow parameters of a lumen, in accordance with some aspects of the present disclosure.
Fig. 4 is a schematic diagram illustrating a heart, and includes an example of a proximal position Posₐ and a distal position Pos_{d} in a lumen 110, in accordance with some aspects of the present disclosure.
Fig. 5 is a schematic diagram illustrating an earlier image and a later image in second temporal sequence of angiographic images A2_{1..n}, in accordance with some aspects of the present disclosure.
Fig. 6 is a schematic diagram illustrating a mapping of the positions of a front of a subsequent contrast agent injection Inj2 in an earlier image and a later image in a second temporal sequence of angiographic images A2_{1..n}, to a common centerline, in accordance with some aspects of the present disclosure.
Fig. 7 is a schematic diagram illustrating an example of a technique for determining a temporal velocity profile V₂(t) of a contrast agent injected into a lumen 110, in accordance with some aspects of the present disclosure.

### Detailed Description

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer implemented method, and in a computer program product, in a corresponding manner.

In the following description, reference is made to a system for determining flow parameters of a lumen. In some examples, flow parameters are determined in a coronary lumen. For example, reference is made to an example of a lumen in the form of the left coronary artery. However, it is to be appreciated that this serves only as an example. The system may be used to determine flow parameters in coronary lumens, as well as in lumens that are found elsewhere in the body. More generally, the system may be used to determine flow parameters of a lumen of the vasculature. The system may be used to determine flow parameters in arteries and veins in the vasculature, including for example the iliac artery, the femoral artery, the popliteal artery, the tibial artery, the pulmonary arteries, systemic arteries.

Reference is also made herein to the use of the system to determine specific flow parameters. Various specific examples refer to flow parameters such as transit velocity, transit time, HMR, IMR, and CFR. Some of the example parameters relate to indices that are computed using a pressure value. However, the use of the system is in general not limited to use in determining these specific parameters, or to determining flow parameters that are dependent on a pressure value, and the system may be used to determine flow parameters in general.

Reference is also made herein to the use of angiographic data. In this respect it is to be appreciated that the X-ray image data may be generated by various types of X-ray imaging systems. By way of an example, the X-ray image data may be generated by the Philips Azurion 7 X-ray imaging system marketed by Philips Healthcare, Best, The Netherlands. The angiographic data may in general be generated by a projection X-ray imaging system that generates 2D X-ray images, or by a volumetric X-ray imaging system that generates volumetric X-ray images. Projection X-ray imaging systems typically include a support arm such as a so-called "C-arm", or an "O-arm", that supports an X-ray source-detector arrangement. Projection X-ray imaging systems may alternatively include a support arm with a different shape to these examples. Projection X-ray imaging systems typically generate 2D X-ray images with the support arm held in a static position with respect to an imaging region during the acquisition of image data. By contrast, volumetric X-ray imaging systems typically generate image data whilst rotating, or stepping, an X-ray source-detector arrangement around an imaging region, and subsequently reconstruct the image data obtained from multiple rotational angles into a volumetric image. Examples of volumetric X-ray imaging systems include computed tomography "CT" imaging systems, cone beam CT "CBCT" imaging systems, and spectral CT imaging systems.

Reference is also made herein to the use of an injector to deliver a contrast agent. In this respect it is to be appreciated that various types of contrast agent injectors may be used. An example of a suitable injector is the Medrad Mark 7 Arterion ^{™} Injection System marketed by Medrad Europe, B.V., Beek, The Netherlands. This, however, is mentioned purely by way of an example.

It is noted that the methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, the Internet, or the Cloud. The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact·disk-read only memory "CD-ROM", compact disk-read/write "CD-R/W", Blu-Ray^{™} and DVD.

As mentioned above, various lumen flow parameters are measured with the lumen in the hyperemic state. A technique for inducing the hyperemic state that involves the injection of a contrast agent, rather than a drug, is published in a document by Shiode, N. et al., entitled "Contrast-induced Hyperemia as an Alternative to Drug-induced Hyperemia in the Evaluation of the Fractional Flow Reserve in Coronary Lesions", Internal Medicine 2017; 56: pages 253-257, 2017. As compared to the use of drugs to induce the hyperemic state, this contrast agent-based technique offers the benefit of a less complicated workflow, and the avoidance of drug-induced complications such as rhythmic complications, and ventricular tachycardia.

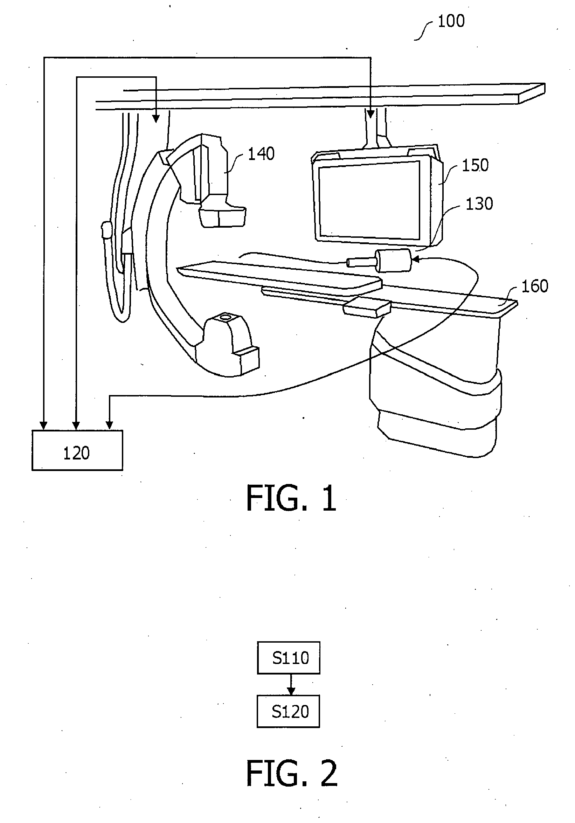

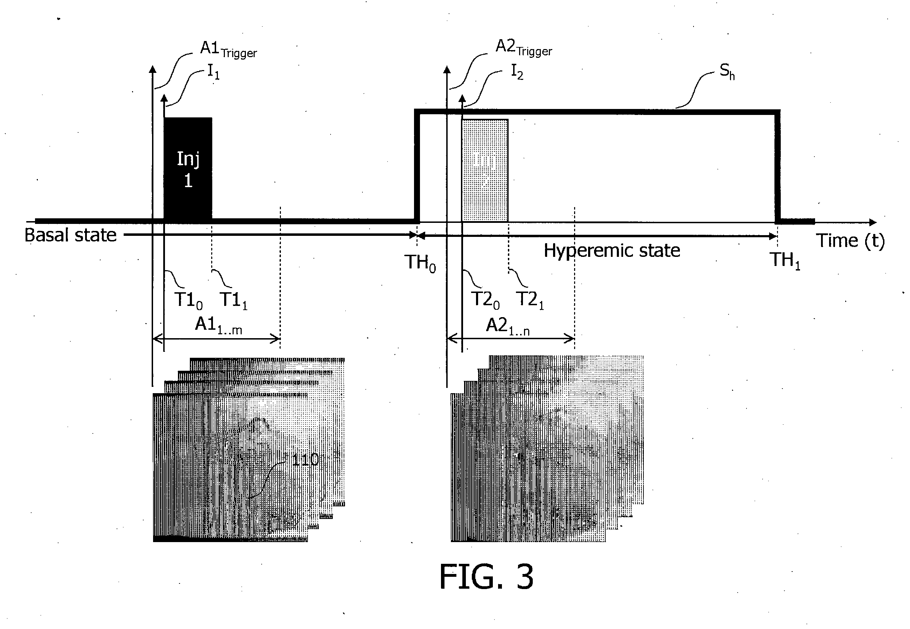

Fig. 1 is a schematic diagram illustrating an example of a system 100 for determining contrast agent flow parameters of a lumen, in accordance with some aspects of the present disclosure. Fig. 2 is a flowchart illustrating an example of a method of determining contrast agent flow parameters of a lumen, in accordance with some aspects of the present disclosure. Operations described in relation to the system 100 illustrated in Fig. 1, may also be performed in the method illustrated in Fig. 2, and vice versa. Fig. 3 is a schematic diagram illustrating an example of various timing signals for determining contrast agent flow parameters of a lumen, in accordance with some aspects of the present disclosure. With reference to Fig. 1 - Fig. 3, the example system 100 is adapted for determining flow parameters of a lumen 110 in a hyperemic state induced subsequent to a contrast agent injection Inj 1 into the lumen in a basal state. The system 100 includes one or more processors 120 configured to:
- determine S110, based on received angiographic data representing the injected contrast agent and/or received injector data representing the injected contrast agent, a temporal window TH₀, TH₁ representing a duration of the induced hyperemic state; and
- output S120 a signal Sₕ indicative of the temporal window TH₀, TH₁.

A lumen in the basal, or resting, state refers to the blood flow when the body is operating at the basal metabolic rate, i.e. when the body is using the amount of energy per unit of time that a person needs to keep the body functioning at rest. A lumen in the hyperemic state refers to the blood flow being increased as compared to the basal state.

In the system 100, the hyperemic state may be induced by the injection of a contrast agent, as described in the document mentioned above by Shiode, N. et al., entitled "Contrast-induced Hyperemia as an Alternative to Drug-induced Hyperemia in the Evaluation of the Fractional Flow Reserve in Coronary Lesions", Internal Medicine 2017; 56: pages 253-257, 2017. The contrast agent may be injected by the contrast agent injector, or simply an "injector" 130 illustrated in Fig. 1. The injector 130 injects the contrast agent in response to a trigger signal, I₁. The trigger signal I₁ is illustrated in Fig. 3, and may be generated manually, or by the processor.120. The injector 130 may include a syringe that contains the contrast agent, as well as a syringe driver that is configured to apply pressure to the syringe in order to inject the contrast agent. The syringe may be fluidically coupled to the lumen via an injection catheter such that when the syringe driver applies pressure to the syringe, the syringe injects the contrast agent through the injection catheter and into the lumen.

In some examples, the hyperemic state is induced from the basal state by injecting a dose of contrast agent into the lumen. The contrast agent may include Iodine, or a Lanthanide such as Gadolinium, or indeed another substance that provides visibility of a flow in the lumen into which the contrast agent is injected. In these examples, the injector 130 may be configured to inject the contrast agent so as to provide a total injected dose of contrast agent. The contrast agent may have a concentration that is measured as a weight per unit of volume, such as milligrams per millilitre, i.e. mg/ml, for example. The injector 130 may be configured to inject the contrast agent at a contrast agent injection rate. The injection rate may be measured as a volume per unit time, such as millilitres per second, ml/s, for example. The total injected dose may be measured in milligrams, mg, or another unit of weight, for example. The injector 130 may be configured to provide a total injected dose of the contrast agent by injecting the contrast agent with a known concentration, at a known injection rate, over a known period of time. The period of time may be brief, for example it may be in the range from a fraction of a second to a few seconds. Such an injection is sometimes referred to as the injection of a "bolus" of contrast agent, or simply an "injected bolus". The injection rate may be constant over time, or it may vary over time. If the injection rate varies over time, the rate may be integrated over time in order to determine the total injected volume of contrast agent, and thus used to determine the total injected dose of the contrast agent.

The processor(s) 130 illustrated in Fig. 1 determines the temporal window TH₀, TH₁ representing a duration of the induced hyperemic state, using received angiographic data representing the injected contrast agent and/or received injector data representing the injected contrast agent. An example of such a temporal window TH₀, TH₁ that is induced consequent to a contrast agent injection Inj 1 into the lumen in the basal state, is illustrated in the upper portion of Fig. 3. In this portion of Fig. 3, the contrast agent injection Inj1 is illustrated by means of a dark-shaded pulse that represents the contrast agent injection rate on the vertical axis, over time, "Time (t)", on the horizontal axis. The contrast agent injection Inj 1 begins at a time T1₀, and ends at a time T1₁.

As mentioned above, the contrast agent injection Inj 1 may be initiated in response to a trigger signal I₁, which is also illustrated in Fig. 3. The trigger signal I₁ may be generated manually, or by the processor 120. The contrast agent injection rate in this example is constant during the period from T1₀ to T1₁, although as mentioned above, the injection rate may alternatively vary over time. After a delay, the hyperemic state begins at the time TH₀. The hyperemic state may in some examples begin after a delay of some seconds. For example the hyperemic state may begin approximately three seconds after the end of the contrast agent injection T1₁. The delay may be measured with respect to the start of the contrast agent injection T1₀, or with respect to the end of the contrast agent injection T1₁. After a further period, the hyperemic state ends at a time TH₁. The hyperemic state may end approximately twelve seconds after the end of the contrast agent injection T1₁. It is noted that whilst the simplified diagram in Fig. 3 illustrates instantaneous transitions between the basal and hyperemic states, this is not necessarily the case in practice. In practice, the transitions between these states at T1₀ and T1₁, may take place over longer periods of time, and therefore the start and the end of this window may be less abrupt.

The time of the onset of the hyperemic window, THo, and its duration, defined by TH₁ - THo, may be affected by one or more factors. These factors may include one or more of: a total injected dose of the contrast agent, a start and/or end time of the contrast agent injection, an intensity profile of the injected contrast agent, a type of the contrast agent, a basal heart rate of the patient, a hyperemic heart rate of the patient, a systolic pressure of the patient, a diastolic pressure of the patient, a gender of the patient, a body mass index of the patient, a clinical condition of the patient.

The dependence of the time of the onset of the hyperemic window, THo, and/or its duration TH₁ - TH₀, on these factors may be determined empirically from measurements on different patient cohorts, and used to provide a physiological model, or a lookup table, that describes the time of the onset of the hyperemic window, THo, and/or its duration TH₁ - TH₀. Similarly, other factors that may also affect the time of the onset of the hyperemic window, THo, and/or its duration TH₁ - TH₀, and these factors may similarly be included in the physiological model or the lookup table. Generally speaking, a simpler physiological model may be provided by using a physiological model that includes only one factor, and a more complex, and potentially more accurate physiological model, may be provided by taking multiple factors into account.

With reference to Fig. 1 - Fig. 3, the one or more factors of the physiological model, or the lookup table, may be determined in the operation S110 using angiographic data representing the injected contrast agent and/or using received injector data representing the injected contrast agent. In the former case, the one or more processors 120 are configured to determine the temporal window TH₀, TH₁ based on received angiographic data. The angiographic data comprises a first temporal sequence of angiographic images A1_{1..m}, and the one or more processors 120 are configured to:
- analyse the first temporal sequence of angiographic images A1_{1..m} to compute at least one of: an end time T1₁ of the contrast agent injection Inj1, a start time T1₀ of the contrast agent injection Inj 1, and an intensity profile of the injected contrast agent; and
- determine the temporal window TH₀, TH₁ based on the computed at least one end time, start time, and intensity profile, respectively.

With reference to the example illustrated in Fig. 1, the processor(s) 120 may receive the angiographic data from the X-ray imaging system 140. As described above with reference to Fig. 3, the angiographic data may be generated.in response to a trigger signal A1_{Trigger}. The trigger signal A1_{Trigger} may be generated manually, or by the processor 120. The angiographic data may be received from various types of X-ray imaging systems, as described above. The angiographic data may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

The angiographic data that is received from the X-ray imaging system 140 may include one or more temporal sequences of X-ray images. The angiographic data represents the injected contrast agent. In some examples, the X-ray images correspond to a portion of the lumen 110 in which the flow parameters are to be determined. In other examples, the X-ray images may correspond to other portions of the anatomy.

The one or more factors of the physiological model, or the lookup table, may be detected from the X-ray images. By way of an example, in the lower left-hand portion of Fig. 3, a -first temporal sequence of angiographic images A1_{1..m}, is illustrated. The X-ray images in this sequence correspond to a portion of the lumen 110. The X-ray images in this sequence may also correspond to the position of a distal end of the injection catheter. A start time T1₀ of the contrast agent injection Inj1 may be detected in X-ray images that correspond to the position of the distal end of the injection catheter by analysing the first temporal sequence of angiographic images A1_{1..m}, and determining the time of the X-ray image in which the contrast agent first appears. Similarly, an end time T1₁ of the contrast agent injection Inj1 may be determined by identifying the time of an X-ray image in the first temporal sequence of angiographic images A1_{1..m}, in which a trailing edge of the injected contrast agent Inj1 starts to move along the lumen 110. Other factors of the physiological model, or the lookup table, may also be determined from the first temporal sequence of angiographic images A1_{1..m}. For example, an intensity profile of the injected contrast agent may be determined by analysing the intensity at a position corresponding to the distal end of the injection catheter in the first temporal sequence of angiographic images A1_{1..m}. In a similar manner, if the first temporal sequence of angiographic images A1_{1..m} corresponds to a portion of the heart, the basal heart rate may be determined from temporal changes in the intensity of these images within the region representing the heart. For example, the position of the coronary arteries may be detected in such images, and the intensity variations representing their movement may be detected in order to provide a signal that varies over time and which represents the heart cycle. The heart rate can then be determined from the heart cycle signal.

Instead of, or in addition to, determining the temporal window THo, TH₁ based on received angiographic data in the operation S110, in some examples injector data is used. In these examples, the one or more processors 120 are configured to determine the temporal window THo, TH₁ based on received injector data. The received injector data represents at least one of: an end time T1₁ of the contrast agent injection Inj1, a start time T1₀ of the contrast agent injection Inj 1, and a total injected dose of the injected contrast agent. The one or more processors 120 are configured to determine the temporal window TH₀, TH₁ based on the computed at least one end time, start time, and total injected dose, respectively.

With reference to Fig. 1, in these examples, the injector data may be received from the injector 130, or from user input device. The injector data may be received from the injector 130 via any form of data communication, including wired, optical, and wireless communication, as described above in relation to the X-ray imaging system 140. Alternatively, the injector data may be received from a user input device such as a keyboard, a mouse, a touchscreen, and so forth. A user may for example read the injector data from a controller of the injector, and manually input the injector data using the user input device. The factors of the physiological model or the lookup table that may be obtained from the injector include: a total injected dose of the contrast agent, a start and/or end time of the contrast agent injection, and an intensity profile of the injected contrast agent.

Irrespective of whether the processor(s) determine the temporal window based on received angiographic data, or based on the received injector data, one or more further factors of the lookup table or the physiological model may be received by the processor 120. These may be received from a sensor that is coupled to the patient, or from a user input device. For example, heart rate data may be received from an electrocardiogram sensor coupled to the patient, and pressure data such as a systolic and diastolic pressure may be received from an extra-corporeal blood pressure monitor or an intravascular blood pressure catheter coupled to the patient. User input data may be received from a user input device, as described above, and used to provide factors such as a type of the contrast agent, and the gender, body mass index, and a clinical condition of the patient.

Thus, the received factor(s) are used as input(s) to the physiological model, or to the lookup table, as described in more detail above, and the temporal window TH₀, TH₁, is determined.

In the operation S120, a signal Sₕ is outputted that is indicative of the temporal window TH₀, TH₁. An example of the signal Sₕ is illustrated via the thick dark line in in Fig. 3, and which illustrates a temporal window having a start time THo, and an end time TH₁. The signal Sₕ may be outputted in various ways, including visually and audially. For example, the signal Sₕ may be outputted to a monitor such as the monitor 150 illustrated in Fig. 1. The signal Sₕ may be outputted in a graphical form, as indicated in Fig. 3, or it may be provided as an optical signal via an indicator lamp that indicates that the lumen is currently in the hyperemic state. In a similar manner, a sound may be provided to indicate that the lumen is currently in the hyperemic state. A countdown corresponding to the start and/or end of the hyperemic state may also be provided to advise an operator of the upcoming start or the end of the hyperemic state. The countdown may be provided in the form of an audio voice that counts down, a sequence of beeps having a frequency that varies in relation to the time remaining until the start or end of the hyperemic state, a flashing indicator lamp, and so forth.

In so doing, the system 100 provides a signal Sₕ that is indicative of the temporal window TH₀, TH₁, thus providing a reliable indication to an operator of when flow parameters of the lumen 110 in the hyperemic state, can be made.

The signal Sₕ that is indicative of the temporal window TH₀, TH₁, may be used for other purposes, as described in more detail in the examples below.

In one example, the one or more processors 120 are configured to output an injector trigger signal I₂ for triggering the injector 130 to perform a subsequent contrast agent injection Inj2 into the lumen 110. The injector trigger signal I₂ is generated within the temporal window THo, TH₁. This example is illustrated in the upper portion of Fig. 3, and wherein the injector trigger signal I₂, and the subsequent contrast agent injection Inj2 are represented. In this portion of Fig. 3, the subsequent contrast agent injection Inj2 is illustrated by means of a light-shaded pulse that represents the contrast agent injection rate on the vertical axis, over time, "Time (t)", on the horizontal axis. The subsequent' contrast agent injection Inj2 has a start time T2₀, and an end time T2₁. The contrast agent injection rate in this example is constant during the period from T2₀ to T2₁, although as mentioned above, the injection rate may alternatively vary over time. The subsequent contrast agent injection Inj2 is also within the temporal window defined by TH₀ and TH₁. The subsequent contrast agent injection Inj2 may be used to generate further angiographic data in order to measure flow parameters of the lumen whilst the lumen is in the hyperemic state.

The injector trigger signal I₂ may be used to automatically trigger the injector 130 to perform the subsequent contrast agent injection Inj2. Thus, the injector trigger signal may be communicated to the injector 130. Alternatively, the injector trigger signal may be outputted in a similar manner to the signal Sₕ indicative of the temporal window TH₀, TH₁, and in response to which an operator manually triggers the subsequent contrast agent injection Inj2. By providing that the injector trigger signal I₂ automatically triggers the injector 130 to perform the subsequent contrast agent injection Inj2, the system 100 provides reliable and repeatable synchronisation of the subsequent injection Inj2 with respect to the hyperemic window TH₀, TH₁. For example, the system 100 may obviate potential timing errors associated with an operator inadvertently delaying the subsequent injection Inj2, and which might result in the need to repeat the subsequent injection Inj2.

The subsequent contrast agent injection Inj2 may be similar in some respects to the contrast agent injection Inj1 described above. Thus, the injector 130 may be controlled in a similar manner to deliver a contrast agent dose in the subsequent contrast agent injection Inj2. The total injected dose that is injected in the subsequent contrast agent injection Inj2 may be the same total injected dose as that which was injected in the contrast agent injection Inj1, or it may be a different total injected dose. The control parameters of the injector 130 that are used for the contrast agent injection Inj1, and for the subsequent contrast agent injection Inj2, may also be the same, or they may be different. For example, the contrast agent injection rates, the duration, and the intensity profiles of the injected doses Inj1 and Inj2 may be the same, or they may be different for the two injections Inj 1 and Inj2.

The contrast agents used in the contrast agent injection Inj1 and the subsequent contrast agent injection, Inj2 may be the same, or they may be different. The injector 130 described above may include one or more syringes that are configured to deliver the contrast agent injection Inj1, and the subsequent contrast agent injection Inj2. When a single syringe is provided, the contrast agent injection Inj 1, and the subsequent contrast agent injection Inj2 are provided by the same syringe, and thus both injections use the same contrast agent. The use of a single syringe has advantages in terms of workflow. The injector 130 described above may alternatively include two or more syringes, and thus separate syringes may be used to deliver the contrast agent injection Inj1, and the subsequent contrast agent injection Inj2. When separate syringes are used, the syringes may couple their contrast agents to the lumen via separate injection catheters, or they may have a shared fluidic path along a single injection catheter that is used to deliver both contrast agents to the lumen 110. The use of different syringes permits the use of different contrast agents in the injections Inj1 and Inj2, as well as the use of a different concentration of the contrast agent in the injections. As compared to the use of a single syringe, the use of multiple syringes therefore offers improved flexibility at the expense of a more complex workflow.

In general, the injection rate of the contrast agent injection Inj 1 that is used to trigger the hyperemic state may be comparable to an average blood flow rate in the lumen in the basal state. For example, it may be within the range of approximately 0.2×V_{pb} and 1.0×V_{pb}, wherein V_{pb} is the average blood flow rate in the basal state. In so doing, the injected contrast agent may substantially replace the blood in the lumen whilst avoiding reflux of the contrast agent towards the aorta. The use of such an injection rate may facilitate the triggering of a relatively long hyperemic window. It also provides a good contrast between the lumen and the background in the image. This injection rate corresponds to the usual injection practice in vascular interventions. However, such an injection rate typically affects the magnitude of the contrast modulation patterns that are created by the mixing of a constant contrast agent injection rate with the pulsatile blood flow in the lumen. If the contrast agent injection rate becomes dominant with respect to the blood flow rate, the contrast dilution modulation effect tends to fade out. Therefore, when a subsequent injection Inj2 is made in order to generate angiographic data in order to measure flow parameters of the lumen, it may be beneficial to use a contrast agent injection rate that is a fraction, such as a tenth, of the average expected hyperemic flow rate in the lumen, i.e. from approximately 0.1×Vₚₕ to 0.3×Vₚₕ wherein Vₚₕ is the average expected blood flow rate in the hyperemic state. Thus, in some examples, the contrast agent injection Inj 1 has a first contrast agent injection rate, and the subsequent contrast agent injection Inj2 has a second contrast agent injection rate, and the second contrast agent injection rate is lower than the first contrast agent injection rate.

The use of a contrast agent injection protocol with differing contrast agent injection rates in the contrast agent injection Inj1, and in the subsequent agent injection Inj2, is not essential, however. This is because the hyperemic state induced by the contrast agent injection Inj1 will in any event give rise to a higher blood flow rate than in the basal state. The higher blood flow rate in the hyperemic state may be sufficient to ensure that the blood flow is not substantially affected by the second contrast agent injection rate of the subsequent contrast agent injection Inj2. Thus, accurate measurements of lumen parameters may also be obtained using contrast agent injection rates that are the same in both the contrast agent injection Inj 1, and in the subsequent agent injection Inj2.

In one example, the processor(s) described with reference to Fig. 1, also generates an imaging trigger signal for triggering the X-ray imaging system 140 to generate further angiographic data. Such a trigger signal is illustrated in Fig. 3 by means of the signal A2_{Trigger}, and which results in the generation of the second, temporal sequence of angiographic images A2_{1..n} illustrated in the lower right-hand portion of Fig. 3. In this example, the processor(s) 120 are configured to determine the temporal window TH₀, TH₁ based on received angiographic data that is generated by the X-ray imaging system 140; and the processor(s) 120 are configured to'output an imaging trigger signal A2_{Trigger} for triggering the X-ray imaging system 140 to generate second angiographic data comprising a second temporal sequence of angiographic images A2_{1..n} representing the subsequent contrast agent injection Inj2 into the lumen 110. The imaging trigger signal A2_{Trigger} is generated within the temporal window TH₀, TH₁.

In this example, the second temporal sequence of angiographic images A2_{1..n} represents the subsequent contrast agent injection Inj2 into the lumen 110. The X-ray imaging system 140 may have a similar, or the same field of view when generating the temporal sequence of angiographic images A1_{1..m}, and the second temporal sequence of angiographic images A2_{1..n}. The second temporal sequence of angiographic images A2_{1..n} may capture the progress of a front of the subsequent contrast agent injection Inj2 as it progresses along a portion of the lumen 110. In so doing, the subsequent contrast agent injection Inj2 may be used to determine flow parameters of the lumen, as described in more detail below.

In this example, the imaging trigger signal A2_{Trigger} is generated within the temporal window TH₀, TH₁. The imaging trigger signal A2_{Trrigger} may also be generated such that the second temporal sequence of angiographic images.A2_{1..n} overlaps a portion of the duration of the subsequent contrast agent injection Inj2. In so doing, the flow of the contrast agent injected in subsequent contrast agent injection Inj2 may be captured in the second temporal sequence of angiographic images A2_{1..n}. A start of the second temporal sequence of angiographic images A2_{1..n} may therefore occur prior to the end time of the T2₁ of the subsequent contrast agent injection Inj2, for example the start of the second temporal sequence of angiographic images A2_{1..n} may occur during the subsequent contrast agent injection Inj2, or it may be coincident with, or occur prior to, the start time T2o of the subsequent contrast agent injection Inj2. The second temporal sequence of angiographic images A2_{1..n} may completely overlap the duration of the subsequent contrast agent injection Inj2. At least some of the images in the second temporal sequence of angiographic images A2_{1..n} may be generated prior to, or after, the subsequent contrast agent injection Inj2.

The use of two separate temporal sequences of X-ray images to capture the flow in this manner has significant benefits in terms of reliability and reduced X-ray dose. In some instances, a front of the contrast agent may traverse a complete lumen of interest within only a few image frames of the second temporal sequence of angiographic images A2_{1..n}. Since in this example the system 100 generates the imaging trigger signal A2_{Trigger} automatically with respect to the subsequent contrast agent injection Inj2, the system 100 reduces the risk of missing these few image frames following a manual triggering of the X-ray imaging system. This improves the reliability of the system 100. Moreover, the system 100 captures the desired flow of the contrast agent with a reduced X-ray dose. An alternative to using separate acquisitions of the X-ray images for each of the contrast agent injection Inj1, and the subsequent contrast agent injection Inj2, might be to continuously capture X-ray images so as to capture the flow resulting from both these injections in a single temporal sequence. However, this would likely require the X-ray images to be generated over a period of some ten seconds or more. This would result in a high X-ray dose, and a significant number of redundant X-ray image frames.

In some examples, the processor(s) 120 receive the second angiographic data; i.e. the second temporal sequence of angiographic images A2_{1..n}. The processor(s) may use this data to determine flow parameters of the lumen 110, as described in more detail below.

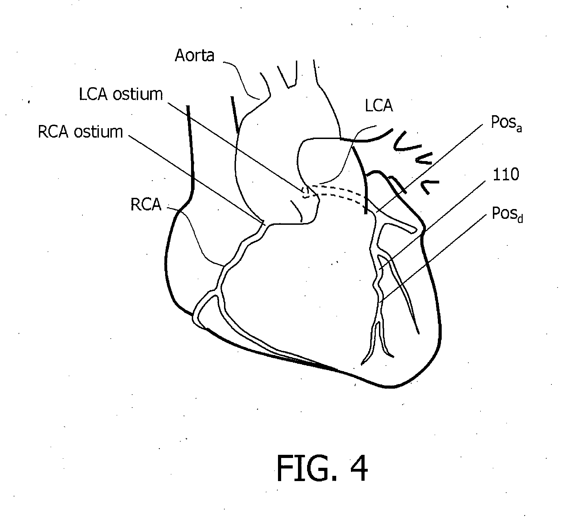

In one example, a distal pressure at a distal position in the lumen is estimated using a measured proximal pressure that is measured at a proximal position in the lumen. The estimate of the distal pressure may be used to compute flow parameters such as the IMR and the HMR. The ability to accurately estimate the distal pressure is useful since it avoids the need to position a pressure sensor in the lumen at the distal position. In this example, it is desired to determine the distal pressure during the hyperemic state. This example is illustrated with reference to Fig. 4, which is a schematic diagram illustrating a heart, and includes an example of a proximal position Posₐ and a distal position Pos_{d} in a lumen 110, in accordance with some aspects of the present disclosure. In the example illustrated in Fig. 4, it is desired to determine the distal pressure at a position Pos_{d} in the left coronary artery, LCA. In this example, the processor(s) 120 are configured to:
- receive pressure sensor data representing a proximal pressure Pa₂ at a proximal position Posₐ in the lumen 110 at a time corresponding to the second temporal sequence of angiographic images A2_{1..n};
- segment one or more images in the first temporal sequence of angiographic images A1_{1..m} to provide a geometric model of the lumen for modelling fluid flow in the lumen 110; and
- estimate a distal pressure P_{d2} at a distal position Pos_{d} in the lumen 110 at the time corresponding to the second temporal sequence of angiographic images A2_{1..n} based on the geometric model and the proximal pressure Pa₂.

Since the distal pressure is estimated using the proximal pressure Pa₂ at a time corresponding to the second temporal sequence of angiographic images A2_{1..n}, the estimated distal pressure is provided for the hyperemic state. It is to be noted that the left coronary artery illustrated in Fig. 4 serves only as an example of a lumen in which a distal pressure is estimated, and that distal, or more generally downstream, pressures in other lumens may be estimated in a similar manner based on a measured proximal, or more generally upstream, pressure.

In general, the terms proximal position and distal position used herein are defined with respect to the ostium of the relevant lumen. The proximal position and distal positions also refer to positions with respect to conventional blood flow in the lumen; the proximal position being relatively upstream with respect to conventional blood flow in the lumen, and the distal position being relatively downstream with respect to conventional blood flow in the lumen. Thus in Fig. 4, the proximal position Posₐ is proximal to the left coronary artery ostium, and the distal position Pos_{d} is distal to the left coronary artery ostium. In the determination of some flow parameters, the distal position may refer more specifically to a position in the "distal two-thirds" of the target lumen, as mentioned in a document by Kobayashi, Y., and Fearon, W. F., entitled "Invasive coronary microcirculation assessment - Current status of Index of Microcirculatory Resistance", Circulation Journal, Official Journal of the Japanese Circulation Society, 2014; 78(5); pages 1021-1028. In the determination of some flow parameters, the distal position is sometimes taken as the most distal position in the lumen in an angiographic image.

In this example, the proximal pressure sensor data may be provided by various types of sensor, including an extra corporeal sensor, and an intraluminal sensor. The injector 130 may include such an extracorporeal pressure sensor, for example. Injectors, such as the injector 130, often include an extracorporeal pressure sensor that is used to monitor the pressure in the lumen during the injection of the contrast agent. The extracorporeal pressure sensor is fluidically coupled to the injection catheter. Since fluid is incompressible, the pressure sensor can measure the intraluminal pressure at the distal end of the injection catheter from an extracorporeal position. Such an extracorporeal pressure sensor may be used to measure the proximal pressure at a proximal position Posₐ that is defined by the position of the distal end of the injector's injection catheter. As an alternative to an extracorporeal sensor, the injection catheter may similarly be provided with an intraluminal pressure sensor on a portion of the injection catheter that is inserted into the lumen. Similarly, other types of intraluminal devices may be present in the lumen and may likewise be used to provide the proximal pressure Pₐ₂ at a proximal position in the lumen, such as a pressure wire, for example.

In this example, the proximal pressure Pa₂ is measured at a time corresponding to the second temporal sequence of angiographic images A2_{1..n}. In this respect, the proximal pressure Pa₂ may be measured at any time within the temporal window TH₀, TH₁ in order to provide an accurate estimate of the distal pressure Pd₂ in the hyperemic state. More specifically, the proximal pressure Pa₂ may be measured within the duration of the second temporal sequence of angiographic images A2_{1..n}. The measured proximal pressure Pₐ₂ may represent an average, instantaneous, or temporal pressure. For example, it may represent an average pressure over the cardiac cycle, or the pressure at a specific point in the cardiac cycle, such as the maximum pressure, the minimum pressure, the systolic pressure, and the diastolic pressure, or indeed the pressure as a function of time over the cardiac cycle.

In this example, one or more images in the first temporal sequence of angiographic images A1_{1..m} are segmented in order to provide a geometric model of the lumen for modelling fluid flow in the lumen 110. Various image segmentation techniques are known for this purpose, including thresholding, template matching, active contour modeling, model-based segmentation, neural networks, e.g., U-Nets, and so forth. The segmentation provides geometric measurements of the lumen 110. The lumen may for example be represented by a 3D centerline with elliptic cross-sections along a length of the 3D centerline. A ID, or a 3D hemodynamic model, which may be based on Navier-Stokes partial differential equations, may then be used with the geometric model to determine the fluid flow in the lumen, and ultimately to estimate the distal pressure P_{d2}. The 3D haemodynamic model may be solved in 3D space using Computational Fluid Dynamics, "CFD" techniques. In this operation it is noted that the segmentation is performed on the first temporal sequence of angiographic images A1_{1..m}. The segmentation is performed on the first temporal sequence of angiographic images A1_{1..m} in order to provide an accurate model of the lumen. As compared to the second temporal sequence of angiographic images A2_{1..n}, the first temporal sequence provides improved visibility of the lumen. This is due to the increased blood flow in the hyperemic state as compared to the basal state, which tends to dilute the contrast agent, and/or the use of a higher total injected dose of the contrast agent in the contrast agent injection Inj1, as compared to in the subsequent contrast agent injection Inj2.

In this example, the geometric model, and the proximal pressure Pₐ₂, are used to estimate a distal pressure P_{d2} at a distal position Pos_{d} in the lumen 110 at the time corresponding to the second temporal sequence of angiographic images A2_{1..n} based on the geometric model and the proximal pressure Pₐ₂. The distal pressure may represent an average, or an instantaneous, or a temporal pressure, as mentioned above in respect of the proximal pressure Pₐ₂ above.

In summary, in this example, the system 100 provides an estimate of the distal pressure Pd₂ in the lumen in the hyperemic state with an angiographic technique that obviates the associated drawbacks of determining flow parameters in angiographic images that have a weak contrast agent. This is achieved by segmenting the angiographic image(s) from the first temporal sequence of angiographic images A1_{1..m} to provide an accurate model of the lumen. The system also obviates the drawbacks associated with using a drug to trigger the hyperemic state because the hyperemic state is triggered by a contrast agent injection. The system also obviates the need to position a pressure sensor at the distal position in the lumen.

The estimate of the distal pressure Pd₂ in the lumen may be used to compute one or more further flow parameters of the lumen, such as the FFR, which is defined as the ratio of the distal pressure to the proximal pressure, i.e. Pd₂/Pa₂, and as described below, other flow parameters such as the IMR and the HMR.

In another example, the first and second temporal sequences of X-ray images A1_{1..m} and A2_{1..n} are used to determine a transit time for the lumen. The transit time, and the associated transit velocity are also examples of flow parameters. The transit time and transit velocity are measures of a lumen's ability to deliver oxygen to surrounding tissues. The transit time is defined as the time taken for blood to flow between a proximal position Posₐ in the lumen and a distal position Pos_{d} in the lumen. When performed in an artery, guidelines state that the ostium of the relevant artery should be chosen as the proximal position. The distal position is less well defined, and it typically reported in literature as a position in the "distal two-thirds" of the target lumen, as mentioned in a document by Kobayashi, Y., and Fearon, W. F., entitled "Invasive coronary microcirculation assessment - Current status of Index of Microcirculatory Resistance", Circulation Journal, Official Journal of the Japanese Circulation Society, 2014; 78(5); pages 1021-1028.

The ability to perform an angiographic measurement of the transit time obviates the need to position flow or velocity sensors in the lumen. Moreover, the ability to measure the transit time by triggering the hyperemic state using a contrast agent obviates the workflow complications and potential patient complications associated with the use of drugs. However, making an angiographic measurement of the transit time can be complex. The need to make angiographic images of the lumen in the hyperemic state often requires a low contrast agent injection rate, as mentioned above. The angiographic images consequently have a weak contrast between the lumen and the background, making it challenging to detect the flow of the contrast agent in the lumen. This challenge is compounded when making transit time measurements in cardiac angiographic images in particular due to the inherent motion of the heart.

With reference to Fig. 3, in this example, the processor 120 receives both the angiographic data, and the second angiographic data. In other words the processor receives both the first temporal sequence of angiographic images A1_{1..m}, and the second temporal sequence of angiographic images A2i..n. The second temporal sequence of angiographic images A2_{1..n} comprises a front of the contrast agent injected in the subsequent contrast agent injection Inj2. Moreover, the processor(s) 120 are configured to:
- analyse one or more images in the first temporal sequence of angiographic images A1_{1..m} to identify a centerline of the lumen 110 in the one or more images;
- identify a centerline of the lumen 110 in at least an earlier image and a later image in the second temporal sequence of angiographic images A2_{1..n} based on a mapping of at least one centerline from the first temporal sequence of angiographic images A1_{1..m} to the earlier image and to the later image; and
- determine a transit time T_{T} taken by the front to pass between a proximal position Posₐ in the lumen 110 in the earlier image, and a distal position Pos_{d} in the lumen 110 in the later image, the transit time being defined by a time difference between the later image and the earlier image.

In the first of these operations, one or more images in the first temporal sequence of angiographic images A1_{1..m} are analysed in order to identify a centerline of the lumen 110. In some instances a single image may be used for this purpose. For example, a single image may be used in which the contrast agent extends between the proximal position Posₐ and the distal position Pos_{d}. Such an image illustrates the portion of the lumen of interest by way of the contrast agent. Moreover, since it is generated from the contrast agent injection Inj 1, it may have a high contrast between the lumen and the background. A centerline of this image is then identified. The centerline may be identified by detecting the lumen by applying a threshold pixel intensity to the image, and determining the centroid positions of groups of pixels across the lumen, at each of multiple positions along the lumen.

In the second of these operations, the centerline that was identified in the image from the first temporal sequence of angiographic images A1_{1..m}, is used to identify the centerline in an earlier image and a later image in the second temporal sequence of angiographic images A2_{1..n}. This operation therefore takes advantage of the typically higher.contrast between the lumen and the background in the first temporal sequence of angiographic images A1_{1..m}, to determine the centerline in the earlier and later images and which may have lower contrast between the lumen and the background.

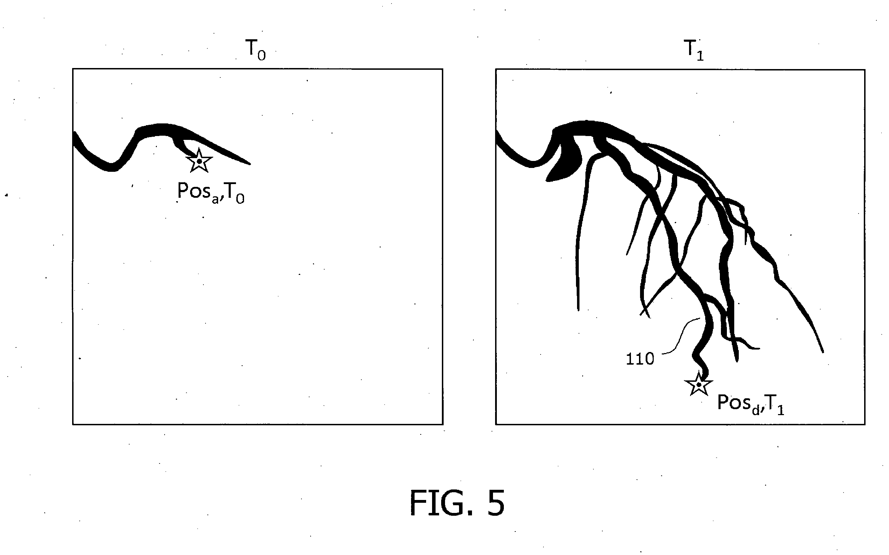

An example of an earlier image and a later image are illustrated in Fig. 5, which is a schematic diagram illustrating an earlier image and a later image in second temporal sequence of angiographic images A2_{1..n}, in accordance with some aspects of the present disclosure. In Fig. 5, the earlier image is shown on the left-hand side, and occurs at time T₀. The later image is shown on the right-hand side, and occurs at time T₁. The angiographic images represent a portion of the cardiac vasculature. The angiographic images illustrate the position of a front of the contrast agent that is injected in the subsequent contrast agent injection Inj2. The angiographic images include a lumen of interest 110 in which it is desired to measure flow parameters. In the earlier image, the front is at a proximal position at time T₀, labelled as Posa,To, and in the later image, the front is at a distal position at time T₁, labelled as Pos_{d},T₁. The schematic images are shown with high contrast between the lumen and the background for illustration purposes. However, it is to be appreciated that in practice the contrast is typically much weaker in these images, and also that the positions of the lumens may also vary between the earlier image and the later image due to cardiac motion.

The centerline of the lumen 110 in the earlier image and the later image, is identified based on a mapping of at least one centerline from the first temporal sequence of angiographic images A1_{1..m} to the earlier image and to the later image. If the lumen is in the same position in each of these images, the centerline may be mapped into the earlier and later images by simply overlaying the centerline on these images. However, if the lumen has moved, for example due to cardiac motion or breathing motion, the centerline may be mapped into each of the earlier and later images by performing a rigid or elastic registration of the centerline until a best fit position of the centerline is identified in the earlier image and the later image. In so doing, a centerline of the lumen 110 is identified in the earlier and later images in the second temporal sequence of angiographic images A2_{1..n}.

In other instances, multiple images in the first temporal sequence of angiographic images A1_{1..m} are used to identify a centerline of the lumen 110 in the earlier image, and in the later image, in a similar manner. The multiple images may correspond to different portions of the cardiac cycle. The multiple images may therefore represent the lumen in different positions. The multiple images may be used to provide a set of centerlines which are used to identify the centerlines of the lumen in each of the earlier and later images.

The first and second operations described above may be implemented by a path finding algorithm, such as Fast-Marching, may be used repetitively to estimate the centerline of a lumen in a first image whilst being constrained by the resemblance (for instance in position, angulation, curvature) to the centerline of the lumen in a second image.

Having identified the centerline of the lumen 110 in the earlier image and the later image, a transit time for the lumen is determined. The transit time T_{T} is the time taken by the front to pass between the proximal position Posₐ in the lumen 110 in the earlier image, and the distal position Pos_{d} in the lumen 110 in the later image. The transit time T_{T} is determined as the time difference between the later image and the earlier image.

In so doing, this example provides an angiographic determination of the transit time of the lumen, whilst obviating the need to position flow or velocity sensors in the lumen, and also obviating the need to use a drug to trigger the hyperemic state.

This example may also be used to determine an average transit time by repeating the determination of the transit time as described above, and computing an average of the transit times. Computing an average transit time in this manner helps to overcome the issue that the transit time is typically only computed over a portion of the cardiac cycle, and over which period the blood velocity in the lumen is not constant.

The transit time that is computed in this example may also be used to determine an IMR value for the lumen 110. Thus, the processor(s) 120 may be configured to compute an index of microcirculatory resistance, IMR, value for the lumen 110, the IMR value being based on a multiplication of the transit time T_{T} and the estimated distal pressure P_{d2}. The estimated distal pressure P_{d2} may be calculated using the model of the lumen that is provided by segmenting the image(s) in the first temporal sequence of angiographic images A1_{1..m}, as described above.

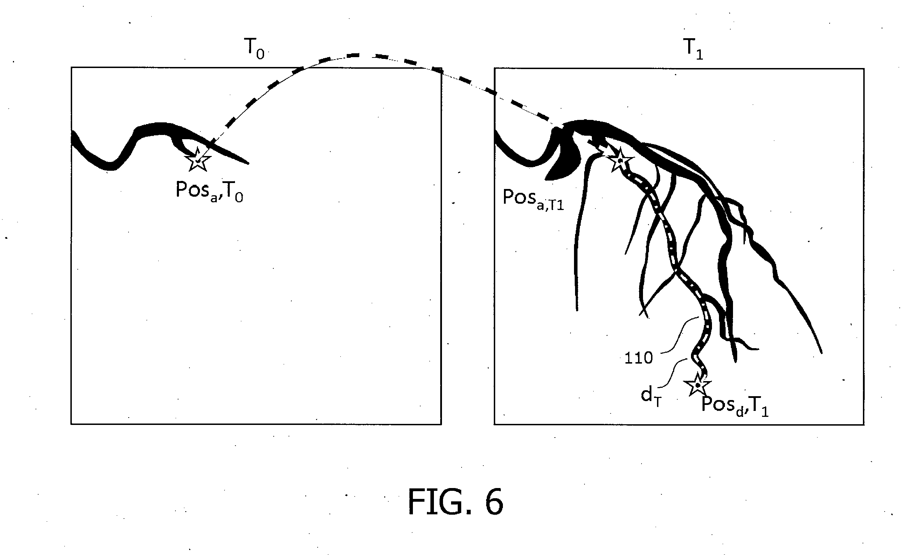

This example may also be used to determine a transit length of the lumen, as now described with reference to Fig. 5 and Fig. 6. The transit length in this example represents the distance travelled by the front between the earlier image and the later image in the second temporal sequence of angiographic images A2i..n. The transit length is useful in determining flow parameters such as the transit velocity, as described in more detail below. Computing the transit length from angiographic images is often complex, particularly when computed from angiographic images of weakly-contrasting lumens in the hyperemic state. Such images may provide an unreliable segmentation. The computation of the transit length is further complicated in the presence of motion between the earlier and later images, as typically occurs in cardiac angiographic images. In the present example, the computation of the transit length in the second temporal sequence of angiographic images A2_{1..n}, is facilitated by using the centerline that was identified in the first temporal sequence of angiographic images A1_{1..m}. As well as facilitating the computation of the transit length, this technique therefore provides improved accuracy and reliability.

In this example, the processor(s) 120 are configured to:
- determine a transit length d_{T} travelled by the front between the proximal position Posₐ in the lumen 110 in the earlier image and the distal position Pos_{d} in the lumen 110 in the later image based on a mapping of the positions of the front in the earlier image and the later image to a common centerline; and
- compute a transit velocity V_{T} for the front based on the ratio of the transit length d_{T} to the transit time T_{T}.

These operations are described with reference to Fig. 5, described above, and Fig. 6, which is a schematic diagram illustrating a mapping of the positions of a front of a subsequent contrast agent injection Inj2 in an earlier image and a later image in a second temporal sequence of angiographic images A2_{1..n}, to a common centerline, in accordance with some aspects of the present disclosure. The images illustrated in Fig. 6 are duplicates of the images in Fig. 5, thus the earlier image is shown on the left-hand side, and occurs at time T₀, and the later image is shown on the right-hand side, and occurs at time T₁. In addition to the features of Fig. 5, Fig. 6 also illustrates an example of a mapping operation by means of the thick dashed line. In the example mapping operation, positions of the front, i.e. Posₐ at time T₀ and Pos_{b} at time T₁, are mapped to the centerline of the lumen 110 in the later image at time T₁. The centerline of the lumen 110 in the later image is illustrated by way of a thin dash-dotted line. Mapping the positions to a common centerline enables the computation of the transit length of the lumen. The common centerline in this example is in the later image. In the later image, the lumen 110 is filled with contrast agent, and therefore the later image provides more detail on the lumen, as compared to the earlier image. In general, the centerline to which the positions Posₐ at time T₀ and Pos_{b} at time T₁ are mapped may however be in any of the images in the first temporal sequence of angiographic images A1_{1..m}, or in the second temporal sequence of angiographic images A2_{1..n}. These centerlines are identified in the operation described above by analysing the image(s) in the first temporal sequence of angiographic images A1_{1..m}, and therefore provide a reliable centerline. Thus, by way of another example, the centerline to which the positions Posₐ at time T₀ and Pos_{b} at time T₁ are mapped may alternatively be an image in the first temporal sequence of angiographic images A1_{1..m} and in which the lumen is filled with contrast agent.

The mapping that is performed in this operation may include transferring the position(s) Posₐ and/or Pos_{b} to the centerline based on a similarity of the shape of the centerline to a shape of the centerline in the image(s) in which the position(s) Posₐ and/or Pos_{b} are determined. Alternatively, the mapping may involve overlaying the earlier and later images if there is negligible motion, or alternatively performing a rigid or elastic registration between the images. Various registration techniques are known for this purpose.

Having mapped the positions Posₐ at time T₀ and Pos_{b} at time T₁ to a common centerline, the transit length d_{T} is computed by calculating the length of the lumen 110 between these two positions. This length may be determined from the angiographic image that includes the mapped positions, using a known calibration of the X-ray imaging system 140. The calibration may provide a scaling between angiographic image pixels in the earlier and later images, and a real-world dimension, such as millimetres or centimetres. Such a calibration may be known for the current geometry of the X-ray imaging system. It may also be computed based on the geometry of the X-ray imaging system 140 using parameters such as the relative separation between the X-ray source, lumen 110, and X-ray detector, and a linear dimension of the X-ray detector. Alternatively, such a calibration may be determined based on the known dimensions of a feature that is included in the angiographic images.

In this example, the transit velocity V_{T} for the front is then determined by calculating the ratio of the transit length d_{T} to the transit time T_{T}.

The transit velocity V_{T} that is computed in this example may also be used to' determine an HMR value for the lumen 110. The HMR is defined as the ratio of the hyperemic distal coronary pressure to the hyperemic mean peak velocity. Thus, processor(s) 120 may be configured to:
- compute a hyperemic microvascular resistance, HMR, value for the lumen 110, the HMR value being based on the ratio of the estimated distal pressure P_{d2} to the computed transit velocity V_{T}.

The estimated distal pressure P_{d2} may be calculated using the model of the lumen that is provided by segmenting the image(s) in the first temporal sequence of angiographic images A1_{1..m}, as described above.

In another example, a temporal velocity profile V₂(t) of the contrast agent injected in the subsequent contrast agent injection Inj2 along a lumen, is determined. The temporal velocity profile is another example of a flow parameter that represents a lumen's ability to transport oxygen between blood and tissue, the flow being determined by the product of the velocity and the cross sectional area of a lumen. The temporal nature of the velocity profile provides additional information on the lumen as compared to a measurement of only a single velocity, such as the average or maximum velocity, or derived quantities such as the pulsatility index, the ratio of diastolic to systolic flow, and so forth. An angiographic technique for determining such a velocity profile in a static lumen is detailed in a document by Pereira V. M., et al. entitled "A DSA-based Method Using Contrast-Motion Estimation for the Assessment of the Intra-Aneurysmal Flow Changes Induced by Flow-Diverter Stents", American Journal of Neuroradiology, 2013, 34(4), pages 808-815. This document discloses the use of the technique to determine a temporal velocity profile in weakly-contrasting angiographic images. In the present example, the technique is adapted further so that the temporal velocity profile can be determined in angiographic images, such as cardiographic angiographic images, in which the lumen typically moves between successive images. In the present example, the presence of such motion is addressed by analysing one or more images in the first temporal sequence of angiographic images A1_{1..m} to identify a centerline of a lumen, and using the centerline(s) to identify the lumen 110 in one or more images in the second temporal sequence of angiographic images A2_{1..n}. Thus, the typically higher contrasting image(s) in the first temporal sequence of angiographic images A1_{1..m} are used to accurately, and reliably, identify the lumen in the second temporal sequence of angiographic images A2_{1..n}. The temporal velocity profile along this lumen can then be determined.

In this example, the processor(s) 120 are configured to:
- analyse one or more images in the first temporal sequence of angiographic images A1_{1..m} to identify a centerline of the lumen in the one or more images;
- identify a lumen 110 in one or more images in the second temporal sequence of angiographic images A2_{1..n} based on a registration of at least one centerline identified in the first temporal sequence of angiographic images A1_{1..m} to the one or more images in the second temporal sequence of angiographic images A2_{1..n};
- determine a temporal velocity profile V₂(t) of the contrast agent injected in the subsequent contrast agent injection Inj2 along the identified lumen 110 in the second temporal sequence of angiographic images A2_{1..n} based on temporal variations in an intensity gradient IG(t) of the contrast agent along the registered centerline in the one or more images in the second temporal sequence of angiographic images A2_{1..n}.

In the first of these operations, the centerline(s) may be determined in one or more images in the second temporal sequence of angiographic images A2_{1..n} using the technique(s) described above. For example, this may include applying a threshold pixel intensity to the image, and determining the centroid positions of groups of pixels across the lumen, at each of multiple positions along the lumen.

In the second of these operations, the lumen may be identified in the image(s) in the second temporal sequence of angiographic images A2_{1..n} by registering the centerline(s) to the image(s) in the second temporal sequence of angiographic images A2i..n. This registration may include determining a similarity of the shapes of the centerlines in the respective images. Various known rigid and elastic registration techniques may be used for this purpose. The registration may include performing a search to determine a best match between the centerline(s) and the lumen in the image(s) in the second temporal sequence of angiographic images A2_{1..n}.

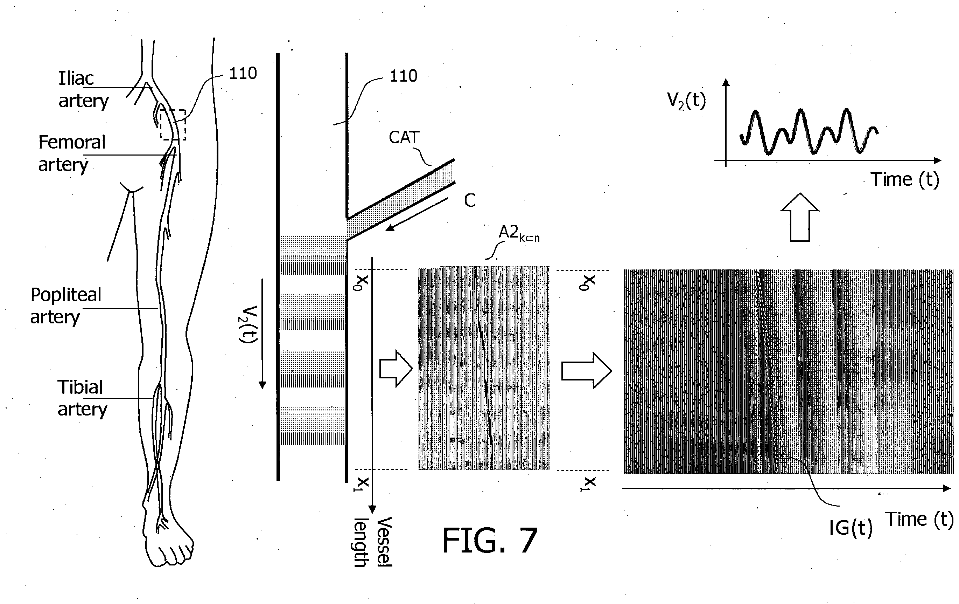

The third of these operations, i.e. determining a temporal velocity profile V₂(t) of the contrast agent, is described with reference to Fig. 7, which is a schematic diagram illustrating an example of a technique for determining a temporal velocity profile V₂(t) of a contrast agent injected into a lumen 110, in accordance with some aspects of the present disclosure. The technique is described in more detail for a static lumen in the document by Pereira V. M., et al. entitled "A DSA-based Method Using Contrast-Motion Estimation for the Assessment of the Intra-Aneurysmal Flow Changes Induced by Flow-Diverter Stents", American Journal of Neuroradiology, 2013, 34(4), pages 808-815. In Fig. 7, the example is given for determining a temporal velocity profile V₂(t) in the femoral artery. However, as mentioned above, the technique is not limited to use in this particular lumen, and may also be used to determine the temporal velocity profile V₂(t) in other lumens, including cardiac lumens and in which motion of the lumen presents additional challenges.

With reference to Fig. 7, starting at the left-hand side of the Figure, a contrast agent, is injected into the lumen 110 by means of a catheter CAT. The contrast agent has a contrast agent injection rate labelled "C" which remains constant during the injection. This injection corresponds to the subsequent contrast agent injection Inj2, and is thus performed when the lumen 110 is in the hyperemic state. As described above, the contrast agent injection rate that is used in the subsequent contrast agent injection Inj2 may be a fraction of the average expected hyperemic flow rate in the lumen, i.e. from approximately 0.1×Vₚₕ to 0.3×Vₚₕ, wherein Vₚₕ is the average expected blood flow rate in the hyperemic state. Consequently, the pulsatile flow of the blood, dilutes the contrast agent in accordance with the blood's temporal velocity. This results in a contrast agent that at any instant in time has an intensity at positions along the lumen that varies in accordance with the blood's temporal velocity at the time of the injection. In Fig. 7, an angiographic image 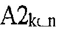 from the second temporal sequence of angiographic images A2_{1..n} captures a profile of the contrast agent distribution along the lumen 110. The profile is indicated in Fig. 7 for positions between x₀ and x₁ in the image 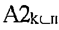 . The profile is then computed along the centerline of the image A2ₖ, the centerline having been provided by the registration operation described above. In the lower right-hand portion of Fig. 7, the profile along the centerline is plotted for each of multiple angiographic images A2i..n, each image corresponding to a vertical line in the illustration in the lower right-hand portion of Fig. 7. An intensity gradient in this Figure, IG(t) may then be computed, and this gradient represents the velocity of a front of the contrast agent as it flows along the lumen. This gradient IG(t), i.e. the velocity of the front, can then be plotted as a function of time, resulting in the temporal velocity profile V₂(t) of the contrast agent illustrated in the upper right-hand portion of Fig. 7.

Determining the temporal velocity profile V₂(t) in this manner allows for the provision of a temporal velocity profile V₂(t) in the presence of a weakly contrasting angiographic images, and also in the presence of lumen motion. Consequently, this example of the system 100 provides a reliable temporal velocity of the blood in the lumen.

The temporal velocity profile V₂(t) described above may be provided for a complete cardiac cycle, thereby enabling a more detailed understanding of the flow in the lumen. Moreover, it may be used to compute further flow parameters for the lumen, such as the HMR. Thus, in this example, the one or more processors 120 may be configured to:
- compute a hyperemic microvascular resistance, HMR, value for the lumen 110, the HMR value being based on the ratio of the estimated distal pressure P_{d2} to the average transit velocity V_{T} from the second angiographic image A2, and wherein the average transit velocity V_{T} is computed as an average of the temporal velocity profile V₂(t) over a complete cardiac cycle.

The estimated distal pressure P_{d2} may be calculated using the model of the lumen that is provided by segmenting the image(s) in the first temporal sequence of angiographic images A1_{1..m}, as described above.

The temporal velocity profile V₂(t) may also be used to improve the estimate of the distal pressure Pd₂ generated by this model. Various haemodynamic models that take account of the temporal velocity profile are known from the field of Computational Flow Dynamics, and may be used for this purpose. In such haemodynamic models, using the temporal velocity profile as an additional boundary condition to the proximal pressure value, typically improves the accuracy of the estimated distal pressure Pd₂. Thus, in this example, the one or more processors 120 may alternatively or additionally be configured to estimate the distal pressure P_{d2} by further inputting the temporal velocity profile V₂(t) into the model.

In another example, the temporal velocity profile V₂(t) is used to compute a haemodynamic index value for the lumen 110. It has been recognised by the inventors that the haemodynamic indices, such as the CFR, and which are based on a comparison between blood velocities in basal and hyperemic states, may be subject to an error. This error arises from measuring these velocity values over only a portion of the cardiac cycle. The error is referred to herein as a phase error, and is typically compensated-for by performing multiple measurements of these erroneous velocity values and computing the average of their values. The repetition of these measurements complicates workflow, and also increases X-ray dose to the patient. In the present example, the phase error is compensated-for by measuring the blood velocity in both the basal and hyperemic states, over the same portion of the cardiac cycle. Consequently, when their ratio is computed to determine haemodynamic indices, such as the CFR, the phase error is eliminated.

In this example, the processor(s) 120 are configured to compute a haemodynamic index value for the lumen 110, wherein the haemodynamic index value is based on a comparison between a fluid velocity in the basal state and a fluid velocity in the hyperemic state. Haemodynamic indices such as the CFR may be computed in accordance with this example. In this example, the one or more processors 120 are configured to determine the fluid velocity in the basal state by:
- analysing the first temporal sequence of angiographic images A1_{1..m} to determine a transit period T_{T1} taken by a front of the injected contrast agent to travel between an upstream position in the lumen and at which a phase in the cardiac cycle has a first phase value Iφ₁, and a downstream position in the lumen and at which the phase in the cardiac cycle as a second value Iφ₂;
- estimating, from the first temporal sequence of angiographic images A1_{1..m}, a transit distance d_{T1} travelled by the front along the lumen 110 between the upstream position and the downstream position; and
- dividing the estimated transit distance d_{T1} by the transit period T_{T1} to provide the fluid velocity in the basal state.

The processor(s) 120 are configured to determine the fluid velocity in the hyperemic state by:
- analysing the temporal velocity profile V₂(t) and the corresponding second temporal sequence of angiographic images A2_{1..n} to identify a time interval starting at a first point in time at which the cardiac phase is equal to the first phase value Iφ₁ in the cardiac cycle, and ending at a second point in time within the same cardiac period at which the cardiac phase is equal to the second phase Iφ₂; and
- calculating the average of the velocity profile V₂(t) over the identified time interval to provide the fluid velocity in the hyperemic state.

In this example, the transit period T_{T1} may be determined in a corresponding manner as the transit time described above. Thus, the upstream position corresponds to a position of the front in an earlier image in the first temporal sequence of angiographic images A1_{1..m}, and the downstream position corresponds to a position of the front in a later image in the first temporal sequence. The transit period T_{T1} corresponds to a time difference between the later image and the earlier image. The upstream and downstream positions represent positions that are proximal and distal to the ostium, respectively. The distal position may for example be in the distal two-thirds of the target lumen.

The phase of the cardiac cycle may be a cardiac phase, i.e. a biologically-defined phase such as the start of the systolic phase, or the start of the diastolic phase, or it may be an angular phase value representing a position in the cardiac cycle in the range 0 to 2π radians. The use of various sensors is contemplated for determining the phase of the cardiac cycle, including electrocardiogram "ECG" sensors, and pressure sensors. Such sensors typically generate signals that include a signature of one or more cardiac phases. The cardiac phase may alternatively be determined from angiographic images. In this respect, it is contemplated to analyse temporal sequence(s) of angiographic images and to detect the pulsation of image features such as the coronary arteries, or the injector's injection catheter, and to derive a signal representing the cardiac phase from these features. For example, a shrinking of the coronary arteries corresponds to the systolic phase, and a dimension of the coronary arteries may be detected in the angiographic images and used to provide a signal indicative of the cardiac phase. In this respect, techniques disclosed in a document by Ciusdel C., et al., entitled "Deep neural networks for ECG-free cardiac phase and end-diastolic frame detection on coronary angiographies", Comput Med Imaging Graph. 2020 Sep;84:101749, may be used.

Thus, the phase in the cardiac cycle may be determined based on received ECG data, or based on pressure data received from an extra-corporeal pressure sensing device coupled to the injector 130, or based on pressure data received from an intraluminal pressure sensing device disposed in the lumen 110, or based on an analysis of the first temporal sequence of angiographic images A1_{1..m}, or based on an analysis of the second temporal sequence of angiographic images A2_{1..n}.

In this example, the transit distance d_{T1} travelled by the front along the lumen 110 between the upstream position and the downstream position, may be estimated by mapping the upstream position of the front from the earlier angiographic image to the later angiographic image with the downstream position of the front, and calculating a length of the lumen between the two positions, as described above in relation to the transit velocity. Thus, the transit distance d_{T1} may be estimated by mapping the upstream position from the earlier image to the later image, or mapping the downstream position from the later image to the earlier image; and determining the transit distance d_{T1} as a length along the lumen 110 between the upstream position and the downstream position in the later image, or in the earlier image, respectively..The first phase value Iφ₁ corresponds to the cardiac phase at the time of the earlier angiographic image, i.e. the angiographic image in the first temporal sequence of angiographic images A1_{1..m} that provides the upstream position. The second phase value Iφ₂ corresponds to the cardiac phase at the time of the later angiographic image, i.e. the angiographic image in the first temporal sequence of angiographic images A1_{1..m} that provides the downstream position.

The temporal velocity profile V2(t), is then analysed to find a time interval that starts at the first phase value Iφ₁, and ends at a second point in time within the same cardiac period at which the cardiac phase is equal to the second phase Iφ₂. Then, the average of the velocity profile V₂(t) is computed over the time interval to provide the fluid velocity in the hyperemic state.

Various haemodynamic indices which are based on a comparison between blood velocities in basal and hyperemic states, such as the CFR, may therefore be calculated from a single injection of contrast agent in each of the basal and hyperemic states whilst eliminating the aforementioned phase error. As compared to the current approach of determining average values for these velocities, the approach in this example simplifies workflow, and also reduces the X-ray dose to a patient because it avoids the need to repeat the measurements multiple times.

With reference to Fig. 1, in some examples, the system 100 described above may also include one or more of an X-ray imaging system 140 for providing angiographic images, an injector 130 for injecting the contrast agent, a monitor 150 for displaying the temporal window Sₕ, a patient bed 160, and a user input device configured to receive user input (not illustrated in Fig. 1), such as a keyboard, a mouse, a touchscreen, and so forth.

The various operations described above in relation to the system 100, may also be provided in the form of a computer-implemented method, or in the form of a computer program product.

Thus, in one example, a computer-implemented method of determining flow. parameters of a lumen 110 in a hyperemic state induced subsequent to a contrast agent injection Inj1 into the lumen in a basal state, is provided. The method includes:
- determining S110, based on received angiographic data representing the injected contrast agent and/or received injector data representing the injected contrast agent, a temporal window TH₀, TH₁ representing a duration of the induced hyperemic state; and
- outputting S120 a signal Sₕ indicative of the temporal window. TH₀, TH₁.

In another example, a computer program product comprising instructions which when executed by one or more processors, cause the one or more processors to carry out a method of determining flow parameters of a lumen 110 in a hyperemic state induced subsequent to a contrast agent injection Inj1 into the lumen in a basal state, is provided. The method includes:
- determining S110, based on received angiographic data representing the injected contrast agent and/or received injector data representing the injected contrast agent, a temporal window THo, TH₁ representing a duration of the induced hyperemic state; and
   outputting S120 a signal Sₕ indicative of the temporal window TH₀, TH₁.

The above examples are to be understood as illustrative of the present disclosure, and not restrictive. Further examples are also contemplated. For instance, the examples described in relation to the system 100, may also be provided by the computer-implemented method, or by the computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for determining flow parameters of a lumen (110) in a hyperemic state induced subsequent to a contrast agent injection (Inj1) into the lumen in a basal state, the system comprising one or more processors (120) configured to:
determine (S110), based on received angiographic data representing the injected contrast agent and/or received injector data representing the injected contrast agent, a temporal window (TH₀, TH₁) representing a duration of the induced hyperemic state; and
output (S120) a signal (Sₕ) indicative of the temporal window (THo, THi).

2. The system according to claim 1, wherein the one or more processors (120) are configured to determine the temporal window (TH₀, TH₁) based on received angiographic data, and wherein the angiographic data comprises a first temporal sequence of angiographic images (A1_{1..m}), and wherein the one or more processors (120) are configured to:
analyse the first temporal sequence of angiographic images (A1_{1..m}) to compute at least one of: an end time (T1₁) of the contrast agent injection (Inj1), a start time (T1₀) of the contrast agent injection (Inj1), and an intensity profile of the injected contrast agent; and
determine the temporal window (THo, THi) based on the computed at least one end time, start time, and intensity profile, respectively.

3. The system according to claim 1, wherein the one or more processors (120) are configured to determine the temporal window (THo, THi) based on received injector data, and wherein the received injector data represents at least one of: an end time (THi) of the contrast agent injection (Inj 1), a start time (T1₀) of the contrast agent injection (Inj1), and a total injected dose of the injected contrast agent; and
wherein the one or more processors (120) are configured to determine the temporal window (THo, THi) based on the computed at least one end time, start time, and total injected dose, respectively.

4. The system according to any one of claims 1 to 3, wherein the one or more processors (120) are further configured to output an injector trigger signal (I₂) for triggering the injector (130) to perform a subsequent contrast agent injection (Inj2) into the lumen (110), and wherein the injector trigger signal (I₂) is generated within the temporal window (THo, TH₁).

5. The system according to claim 4, wherein the contrast agent injection (Inj1) comprises a first contrast agent injection rate, and wherein the subsequent contrast agent injection (Inj2) comprises a second contrast agent injection rate, and wherein the second contrast agent injection rate is lower than the first contrast agent injection rate.

6. The system according to claim 4 or claim 5, wherein the one or more processors (120) are configured to determine the temporal window (TH₀, TH₁) based on received angiographic data;
wherein the angiographic data is generated by an X-ray imaging system (140); and
wherein the one or more processors (120) are further configured to output an imaging trigger signal (A2_{Trigger}) for triggering the X-ray imaging system (140) to generate second angiographic data comprising a second temporal sequence of angiographic images (A2_{1..n}) representing the subsequent contrast agent injection (Inj2) into the lumen (110), and wherein the imaging trigger signal (A2_{Trigger}) is generated within the temporal window (THo, THi).

7. The system according to claim 6, wherein the one or more processors (120) are further configured to receive the second angiographic data.

8. The system according to claim 7, wherein the one or more processors (120) are further configured to:
receive pressure sensor data representing a proximal pressure (Pa₂) at a proximal position (Posₐ) in the lumen (110) at a time corresponding to the second temporal sequence of angiographic images (A2_{1..n});
segment one or more images in the first temporal sequence of angiographic images (A1_{1..m}) to provide a geometric model of the lumen for modelling fluid flow in the lumen (110); and
estimate a distal pressure (P_{d2}) at a distal position (Pos_{d}) in the lumen (110) at the time corresponding to the second temporal sequence of angiographic images (A2_{1..n}) based on the geometric model and the proximal pressure (Pₐ₂).

9. The system according to claim 7 or claim 8, wherein the second temporal sequence of angiographic images (A2_{1..n}) comprises a front of the contrast agent injected in the subsequent contrast agent injection (Inj2), and wherein the one or more processors (120) are further configured to:
analyse one or more images in the first temporal sequence of angiographic images (A1_{1..m}) to identify a centerline of the lumen (110) in the one or more images;
identify a centerline of the lumen (110) in at least an earlier image and a later image in the second temporal sequence of angiographic images (A2_{1..n}) based on a mapping of at least one centerline from the first temporal sequence of angiographic images (A1_{1..m}) to the earlier image and to the later image; and
determine a transit time (T_{T}) taken by the front to pass between a proximal position (Posₐ) in the lumen (110) in the earlier image, and a distal position (Pos_{d}) in the lumen (110) in the later image, the transit time being defined by a time difference between the later image and the earlier image.

10. The system according to claim 9, wherein the one or more processors (120) are further configured to:
• determine a transit length (d_{T}) travelled by the front between the proximal position (Posₐ) in the lumen (110) in the earlier image and the distal position (Pos_{d}) in the lumen (110) in the later image based on a mapping of the positions of the front in the earlier image and the later image to a common centerline; and
compute a transit velocity (V_{T}) for the front based on the ratio of the transit length (d_{T}) to the transit time (T_{T}).

11. The system according to claim 8 or claim 9, wherein the one or more processors (120) are further configured to:
compute an index of microcirculatory resistance, IMR, value for the lumen (110), the IMR value being based on a multiplication of the transit time (T_{T}) and the estimated distal pressure (Pd2).

12. The system according to claim 10, wherein the one or more processors (120) are further configured to:
compute a hyperemic microvascular resistance, HMR, value for the lumen (110), the HMR value being based on the ratio of the estimated distal pressure (P_{d2}) to the computed transit velocity (V_{T}).

13. The system according to claim 7 or claim 8, wherein the one or more processors (120) are further configured to:
analyse one or more images in the first temporal sequence of angiographic images (A1_{1..m}) to identify a centerline of the lumen in the one or more images;
identify a lumen (110) in one or more images in the second temporal sequence of angiographic images (A2_{1..n}) based on a registration of at least one centerline identified in the first temporal sequence of angiographic images (A1_{1..m}) to the one or more images in the second temporal sequence of angiographic images (A2_{1..n});
determine a temporal velocity profile (V₂(t)) of the contrast agent injected in the subsequent contrast agent injection (Inj2) along the identified lumen (110) in the second temporal sequence of angiographic images (A2_{1..n}) based on temporal variations in an intensity gradient (IG(t)) of the contrast agent along the registered centerline in the one or more images in the second temporal sequence of angiographic images (A2_{1..n}).

14. The system according to claim 13, wherein the one or more processors (120) are further configured to:
compute a hyperemic microvascular resistance, HMR, value for the lumen (110), the HMR value being based on the ratio of the estimated distal pressure (P_{d2}) as determined in claim 8 to the average transit velocity (V_{T}) from the second angiographic image (A2), and wherein the average transit velocity (V_{T}) is computed as an average of the temporal velocity profile (V₂(t)) as determined in claim 13 over a complete cardiac cycle; and/or
estimate the distal pressure (P_{d2}) in accordance with claim 8 by further inputting the temporal velocity profile (V₂(t)) into the model.

15. The system according to claim 13, wherein the one or more processors (120) are further configured to compute a haemodynamic index value for the lumen (110), wherein the haemodynamic index value is based on a comparison between a fluid velocity in the basal state and a fluid velocity in the hyperemic state;
wherein the one or more processors (120) are configured to determine the fluid velocity in the basal state by:
analysing the first temporal sequence of angiographic images (A1_{1..m}) to determine a transit period (T_{T1}) taken by a front of the injected contrast agent to travel between an upstream position in the lumen and at which a phase in the cardiac cycle has a first phase value (Iφ₁), and a downstream position in the lumen and at which the phase in the cardiac cycle has a second value Iφ₂;
estimating, from the first temporal sequence of angiographic images (A1_{1..m}), a transit distance (d_{T1}) travelled by the front along the lumen (110) between the upstream position and the downstream position; and
dividing the estimated transit distance (d_{T1}) by the transit period (T_{T1}) to provide the fluid velocity in the basal state; and
wherein the one or more processors (120) are configured to determine the fluid velocity in the hyperemic state by:
analysing the temporal velocity profile (V₂(t)) and the corresponding second temporal sequence of angiographic images (A2_{1..n}) to identify a time interval starting at a first point in time at which the cardiac phase is equal to the first phase value (Iφ₁) in the cardiac cycle, and ending at a second point in time within the same cardiac period at which the cardiac phase is equal to the second phase Iφ₂; and
calculating the average of the velocity profile (V₂(t)) over the identified time interval to provide the fluid velocity in the hyperemic state.
